# EUROPEAN PATENT SPECIFICATION

(11) **EP 2 497 720 B1**
(45) Date of publication and mention of the grant of the patent: **08.08.2018**
(21) Application number: 10828411.8
(22) Date of filing: 05.11.2010
(51) Int. Cl.: B65D 51/18, A61J 1/10, A61J 1/06, A61M 5/24, B65D 51/00

(54) **CARTRIDGE CAP**
KARTUSCHENKAPPE
CAPUCHON DE CARTOUCHE

(30) Priority: 06.11.2009 JP 2009255341
(43) Date of publication of application: 12.09.2012
(73) Proprietor: Daikyo Seiko, LTD., Sano-shi Tochigi 327-0813 (JP)
(72) Inventor: KAWAMURA, Hideaki, Tochigi, 327-081 (JP)
(74) Representative: TBK
(86) International application number: PCT/JP2010/070155
(87) International publication number: WO 2011/055853

(56) References cited:
- WO-A1-94/00349
- WO-A2-2005/000703
- JP-A- H08 276 952
- JP-A- 2000 237 310
- US-A- 3 927 798
- US-A- 4 815 619
- US-A- 5 085 332
- US-A- 5 303 835
- US-A1- 2005 082 305
- US-B1- 7 419 066

## Description

The present invention relates to a cartridge cap according to the preamble of claim 1, more specifically to a cartridge cap for sealing the opening portion of a cylindrical cartridge in which medicinal solution is filled.

### Background Art

It is generally known to use a prefilled syringe in which medicinal solution is filled as a conventional syringe for an injector (see JP 2008-307237 A). As an alternative to this kind of prefilled syringe, it is also generally known to use a conventional type of syringe which loads a prefilled cartridge in which medicinal solution is filled.

In this type of prefilled cartridge, the opening portion of a cylindrical cartridge in which medicinal solution is filled is sealed by a cartridge cap. The cylindrical cartridge incorporates a piston which is connected to a plunger. A gasket is attached to the cartridge cap, a double-ended injection needle is attached to a syringe which loads a prefilled cartridge, and the double-ended injection needle passes through the gasket.

WO 94/00349 A1 shows a cartridge cap according to the preamble of claim 1 for sealing the opening portion of a cylindrical cartridge in which medicinal solution is filled, wherein a plurality of terrace shaped receiving portions are formed on the inner periphery of a cylindrical wall portion in a circumferential direction at predetermined intervals, the cylindrical wall portion continues to a top plate portion, the terrace shaped receiving portion has a joint receiving surface for jointing with the outer periphery of a flange shaped lip portion formed at the opening portion of the cylindrical cartridge and a tucking portion for holding the lip portion between the top plate portion and the tucking portion, and an air relief passage is formed along the inner periphery of the cylindrical wall portion between the terrace shaped receiving portions.

Further cartridge caps according to the prior art are shown in WO 2005/000703 A2 and JP H08-276952 A.

In conventional cartridge caps, it has been frequently observed that air stays in a cylindrical cartridge because there is no escape for the air which was sucked in during a sealing operation of the opening portion of a cylindrical cartridge in which medicinal solution is filled.

### Summary of the Invention

It is the object of the present invention to further develop a cartridge cap according to the preamble of claim 1 such that an improved cartridge cap constitution is provided, which can prevent air from staying in a cylindrical cartridge even if the air is sucked in the cylindrical cartridge during a sealing operation of the opening of the cylindrical cartridge in which medicinal solution is filled, and which has an increased strength of a top plate portion of the cartridge cap.

The object of the present invention is achieved by a cartridge cap having the features of claim 1.

Further advantageous developments of the present invention are defined in the dependent claims.

According to an aspect of the present invention, a cartridge cap is constituted as a cartridge cap for sealing the opening of a cylindrical cartridge in which medicinal solution is filled, wherein a plurality of terrace shaped receiving portions are formed on the inner periphery of a cylindrical wall portion in a circumferential direction at predetermined intervals, the cylindrical wall portion continues to a top plate portion , the terrace shaped receiving portion has a joint receiving surface for jointing with the outer periphery of a flange shaped lip portion which is formed at the opening portion of the cylindrical cartridge and a tucking portion for holding the lip portion between the top plate portion and the tucking portion, and an air relief passage is formed along the inner periphery of the cylindrical wall portion between the terrace shaped receiving portions.

Here, even if air is sucked in during a sealing operation of the opening portion of the cylindrical cartridge in which medicinal solution is filled, the sucked air is let out to the outside from the gap between the outer periphery of the lip portion of the cylindrical cartridge and the inner periphery of the cylindrical wall portion of the cartridge cap through the air relief passage which is formed along the inner periphery of the cylindrical wall portion.

Furthermore, according to the invention, either a plurality of thick portions are formed on the outer surface of the top plate portion in a circumferential direction at predetermined intervals and set the circumferential position of each thick portion so as to correspond to the circumferential position of each air relief passage between the terrace shaped receiving portions because the strength of each portion of the cartridge caps between the terrace shaped receiving portions increases is increased.

In cartridge caps according to the invention, it is preferable to form a tucking portion which is formed on the inner periphery of the cylindrical wall portion in a belt shape which extends in a circumferential direction of the cylindrical wall portion and form a tapered surface on the periphery to guide the outer periphery of the lip portion of the cylindrical cartridge to the holding position because the lip portion can be smoothly guided to the holding position and the opening portion of the cylindrical cartridge can be firmly sealed.

### Effect of Invention

In the cartridge cap according to the invention, even if air is sucked in during a sealing operation of the opening of the cylindrical cartridge in which medicinal solution is filled, the sucked air is let out to the outside from the gap between the outer periphery of the lip portion of the cylindrical cartridge and the inner periphery of the cylindrical wall portion of the cartridge cap through the air relief passage which is formed along the inner periphery of the cylindrical wall portion. Therefore, , it is possible to prevent air from staying in the cylindrical cartridge even if the air is sucked in during a sealing operation of the opening of the cylindrical cartridge in which medicinal solution is filled.

In the cartridge cap according to the invention, the strength of each portion between the terrace shaped receiving portions is increased by thick portions if a plurality of thick portions are formed on the outer surface of the top plate portion in a circumferential direction at predetermined intervals and the circumferential position of each thick portion is set so as to correspond to the circumferential position of each air relief passage between the terrace shaped receiving portions.

In the cartridge cap according to the invention, even if air is sucked in during a sealing operation of the opening of the cylindrical cartridge in which medicinal solution is filled, the sucked air is let out to the outside from the gap between the outer periphery of the lip portion of the cylindrical cartridge and the inner periphery of the cylindrical wall portion of the cartridge cap through the air relief hole which goes through the cylindrical portion. Therefore, it is possible to prevent air from staying in the cylindrical cartridge even if the air is sucked in during a sealing operation of the opening of the cylindrical cartridge in which medicinal solution is filled.

Furthermore, it is possible to seal the opening portion of the cylindrical cartridge more firmly because a belt shaped receiving portion is formed continuously in a circumferential direction.

It is also possible to guide the lip portion to the holding position smoothly and seal the opening portion of the cylindrical cartridge firmly if a tucking portion is formed on the inner periphery of the cylindrical wall portion in a belt shape which extends in a circumferential direction of the cylindrical wall portion and a tapered surface is formed on the periphery to guide the outer periphery of the lip portion of the cylindrical cartridge to the holding position.

### Brief Description of Drawings

Fig. 1 shows a front view of a cartridge cap according a first embodiment of the invention together with a cylindrical cartridge.
Fig. 2 shows a knock down front view of a syringe which shows an example of using a prefilled cartridge having a cylindrical cartridge and a cartridge cap shown in Fig. 1.
Fig. 3 shows an enlarged front view of a cartridge cap shown in Fig. 1.
Fig. 4 shows a front view of a cartridge cap shown in Fig. 3.
Fig. 5 shows a cross sectional view of a cartridge cap along V-V line in Fig. 4.
Fig. 6 shows a cross sectional view of a condition in which a cartridge cap according to a first embodiment sealed the opening portion of a cylindrical cartridge, and shows a cross sectional view along V-V line in Fig. 4.
Fig. 7 shows a cross sectional view of a condition in which a cartridge cap according to a first embodiment sealed the opening portion of a cylindrical cartridge, and shows a cross sectional view along VII-VII line in Fig. 4.
Fig. 8 shows a cross sectional view of a cartridge cap according to a second embodiment of the invention, and this drawing corresponds to Fig. 5.
Fig. 9 shows a cross sectional view of a cartridge cap according to a second embodiment, and shows a cross sectional view along a line perpendicular to V-V line in Fig. 4.
Fig. 10 shows a cross sectional view of a condition in which a cartridge cap according to a second embodiment sealed the opening portion of a cylindrical cartridge, and this drawing corresponds to Fig. 6.

### A mode for implementing the Invention

A cartridge cap according to the first embodiment and a cartridge cap according to the second embodiment will be explained below by referring to the attached drawings.

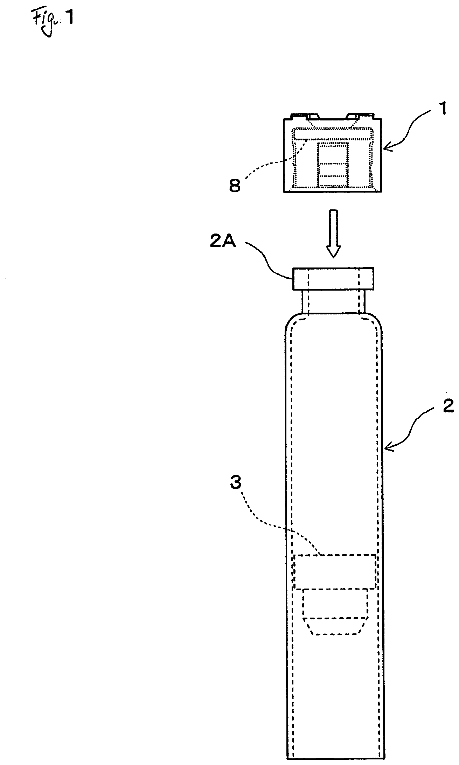

First, a cartridge cap according to a first embodiment will be described by referring to Fig. 1-Fig. 7. As shown in Fig. 1, a cartridge cap 1 according to a first embodiment is used for sealing the opening portion of a cylindrical cartridge 2 in which medicinal solution is filled. The cartridge cap 1 is to fit to a flange shaped circular lip portion 2A which is formed at the opening portion of the cylindrical cartridge 2 by using a capper which is not shown in the drawing.

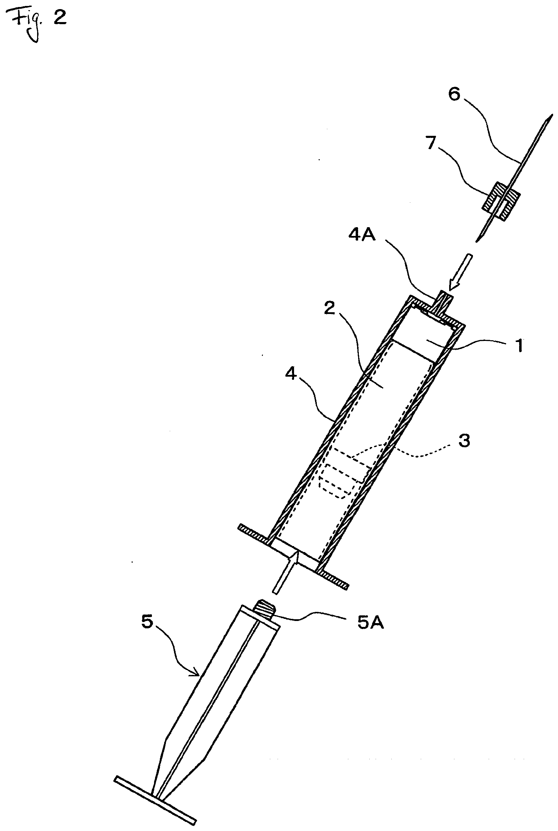

A piston 3 is incorporated in the cylindrical cartridge 2 for filling medicinal solution and pushing out the filled medicinal solution. The cylindrical cartridge 2 is loaded in a syringe 4 of an injector shown in Fig. 2 as a prefilled cartridge by fitting a cartridge cap 1 to the circular lip portion 2A. A connecting screw portion 5A of the tip portion of a plunger 5 is screwed in and connected to the piston 3 in the cylindrical cartridge 2.

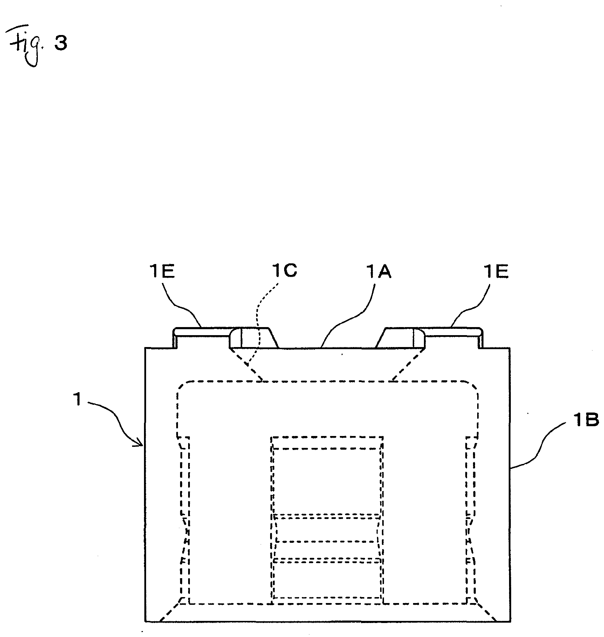

A double-ended injection needle 6 is attached to a needle attaching portion 4A at the tip portion of the syringe 4 through a holder 7. The inner end portion of the double-ended injection needle 6 is inserted into the medicinal solution which is filled in the cylindrical cartridge 2 by passing through the top plate portion of the cartridge cap 1.
As shown in Fig. 3 - Fig. 5, the cartridge cap 1 according to the first embodiment has a cylindrical wall portion 1B which continues to the top plate portion 1A and the center of the top plate portion 1A has a through hole 1D which has a tapered wall surface 1C with a larger diameter at the upper portion. Four thick portions 1E, 1E,... are formed on the outer surface of the top plate portion 1A surrounding the through hole 1D and function as spacers in the syringe 4 shown in Fig. 2. The thick portions 1E, 1E,... are equiangularly positioned in a circumferential direction of the top plate portion 1A at 90 degree intervals.

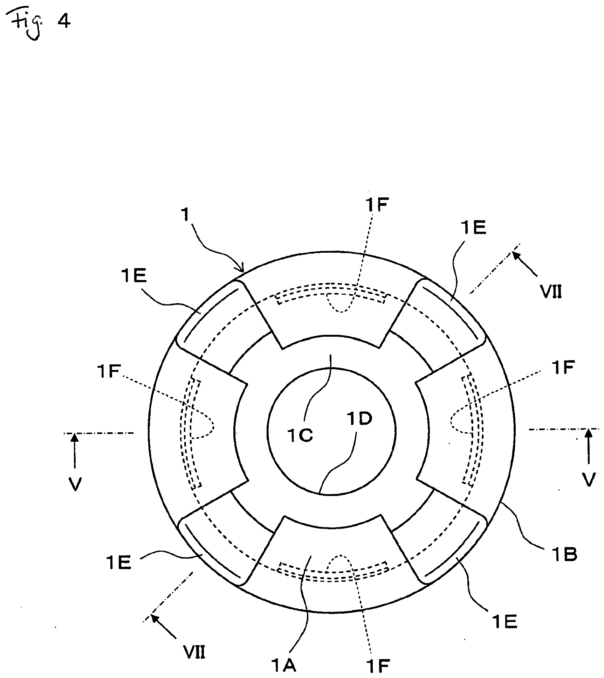

As shown in Fig. 4, four thin terrace shaped receiving portions 1F, 1F,.. are formed on the inner periphery of the cylindrical wall portion 1B of the cartridge cap 1. The thin terrace shaped receiving portions 1F, 1F,.. are equiangularly positioned in a circumferential direction of the cylindrical wall portion 1B at 90 degree intervals and are positioned by shifting a phase by 45 degrees in a circumferential direction with respect to the thick portions 1E, 1E,... which are formed on the outer surface of the top plate portion 1A.

That is to say, each terrace shaped receiver portion 1F is positioned between the thick portions 1E, 1E in a circumferential direction of the cylindrical wall portion 1B.

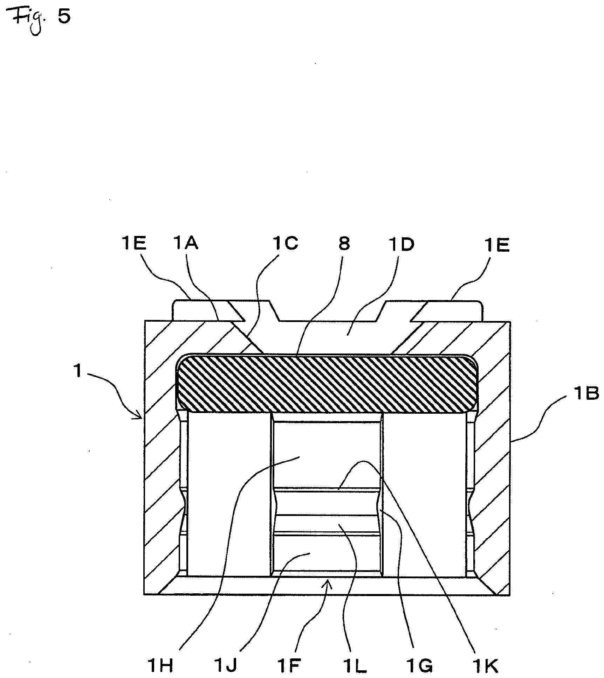

As shown in Fig. 5, each terrace shaped receiving portion 1F has a roughly vertically long rectangle shape from an anterior view. A tucking portion 1G is formed in a belt shape along the circumferential direction of the cylindrical wall portion 1B for locking the lower edge of the circular lip portion 2A of the cylindrical cartridge 2 shown in Fig. 1 and holding the lower edge of the circular lip portion 2A between the top plate portion 1A and the tucking portion 1G. A joint receiving surface 1H is formed on the portion on the upper side of the tucking portion 1G of the terrace shaped receiving portion 1F for jointing with the outer periphery of the circular lip portion 2A of the cylindrical cartridge 2. A guiding surface 1J is formed on the portion on the lower side of the tucking portion 1G of the terrace shaped receiving portion 1F for guiding the outer periphery of the circular lip portion 2A.

The tucking portion 1G of the terrace shaped receiving portion 1F has a shelving angle section and bulges from the joint receiving surface 1H and the guiding surface 1J. A locking portion 1K is formed at the lower edge of the joint receiving surface 1H for locking the lower edge of the circular lip portion 2A(see Fig. 1) of the cylindrical cartridge 2. A tapered guiding surface 1L is formed at the lower half portion of the tucking portion 1G for guiding the outer periphery of the circular lip portion 2A toward the side of locking portion 1K.

The cartridge cap 1 according to the first embodiment configured as described above is used to seal the opening portion of the cylindrical cartridge 2 in which medicinal solution is filled. Prior to the sealing operation, a gasket 8 is loaded in the cartridge cap 1 in advance for sealing the through hole 1D by contacting with the inner surface of the top plate portion 1A as shown in Fig.5. In this condition, the cartridge cap 1 is fit to the circular lip portion 2A which is formed at the opening portion of the cylindrical cartridge 2 by using a capper which is not shown in the drawing.

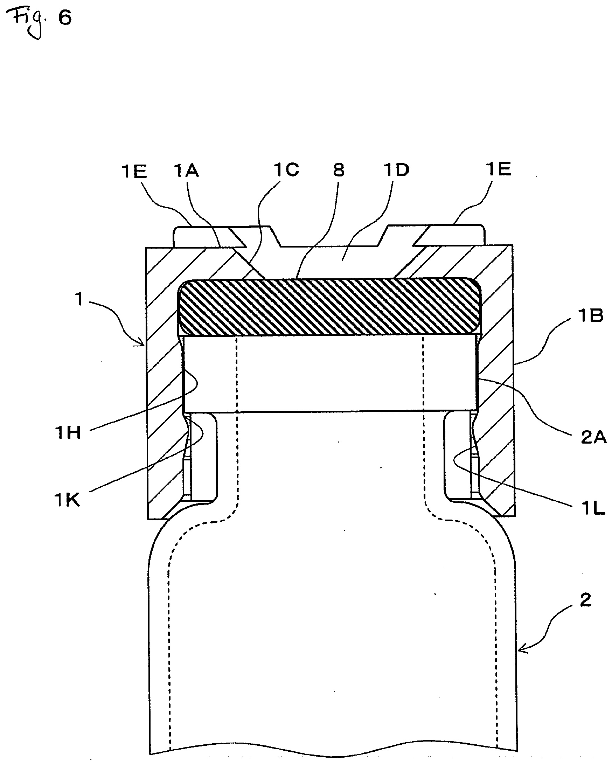

By this capping operation, the cartridge cap 1 is fit to the head portion including the circular lip portion 2A of the cylindrical cartridge 2 as shown in Fig.6 and seals the opening portion of the cylindrical cartridge 2 by the gasket 8 in this condition. That is to say, the cartridge cap 1 seals the opening portion of the cylindrical cartridge 2 by holding the circular lip portion 2A between the locking portion 1K of the four terrace shaped receiving portions 1F, 1F,.. which are formed on the inner periphery of the cylindrical wall portion 1B and the inner surface of the top plate portion 1A through the gasket 8.

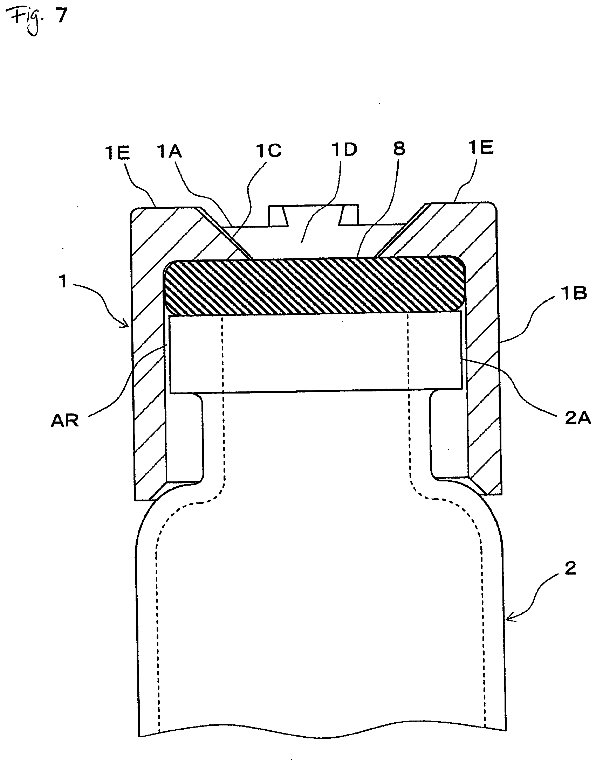

The air which is sucked in the space between the cartridge cap 1 and the opening portion of the cylindrical cartridge 2 during a capping operation is released to the outside of the cylindrical cartridge 2 through an air relief passage AR which is formed along the inner periphery of the cylindrical wall portion 1B including the gap between the outer periphery of the circular lip portion 2A of the cylindrical cartridge 2 and the part of the inner periphery of the cylindrical wall portion 1B of the cartridge cap 1 where no terrace shaped receiving portions 1F, 1F,.. are formed, as shown in Fig. 7.

Therefore, in the cartridge cap 1 according to the first embodiment, it is possible to prevent air from staying in the cylindrical cartridge 2 even if the air is sucked in during a sealing operation of the opening portion of the cylindrical cartridge 2 in which medicinal solution is filled.

Additionally, in the cartridge cap 1 according to the first embodiment, it is possible to increase the strength of the part of the cylindrical wall portion 1B where no terrace shaped receiving portions 1F, 1F,.. are formed by forming the thick portion 1E, 1E,.. because the four thick portions 1E, 1E,... which are formed on the outer periphery of the top plate portion 1A and the four terrace shaped receiving portions 1F, 1F,... which are formed on the inner periphery of the cylindrical wall portion 1B are positioned in a circumferential direction of the cartridge cap 1 by shifting a phase by 45 degrees and each thick potion 1E is positioned so as to correspond to the air relief passage AR which extends along the inner periphery between two terrace shaped receiving portions 1F, 1F of the cylindrical wall portion 1B as shown in Fig. 4,

In a cartridge cap 1 according to the first embodiment, it is possible to firmly seal the opening portion of the cylindrical cartridge 2 by smoothly guiding the circular lip portion 2A to the holding position in a sealing operation of the opening portion of the cylindrical cartridge 2, because the tucking portion 1G of each terrace shaped receiving portion 1F which is formed on the inner periphery of the cylindrical wall portion 1B is formed in a belt shape which extends in a circumferential direction of the cylindrical wall portion 1B and the tapered guide surface 1L is formed on the periphery for guiding the outer periphery of the circular lip portion 2A of the cylindrical cartridge 2 toward the side of the locking portion 1K which corresponds to the holding position.

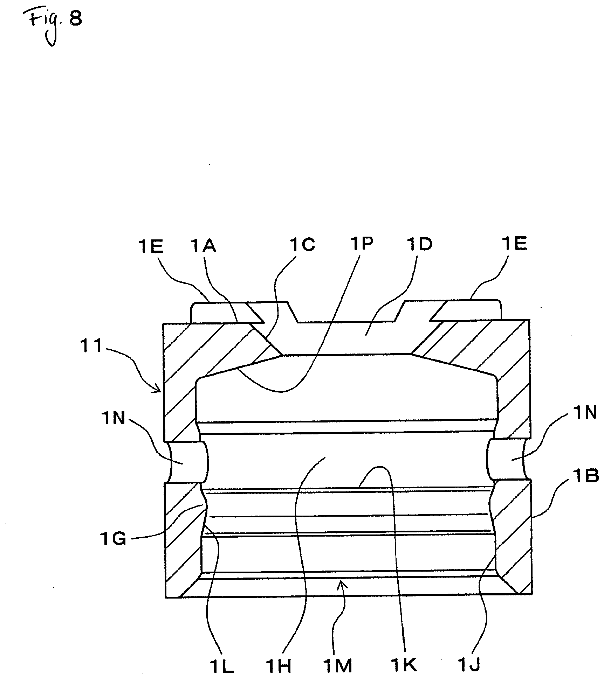

Next, a cartridge cap according to a second embodiment will be described referring to Fig. 8-Fig. 10. Similarly to the first embodiment (see Fig. 1), a cartridge cap according to the second embodiment is used for sealing the opening portion of a cylindrical cartridge 2 in which medicinal solution is filled and the cap 1 is to fit to a flange shaped circular lip portion 2A which is formed at the opening portion of the cylindrical cartridge 2 by using a capper which is not shown in the drawing.

Because the cartridge cap 11 according to the second embodiment has structural portions which are identical to those in the cartridge cap 1 according to the first embodiment, the similarly configured portions will not be described in detail by using the same reference numerals as those in the cartridge cap 1 according to the first embodiment.

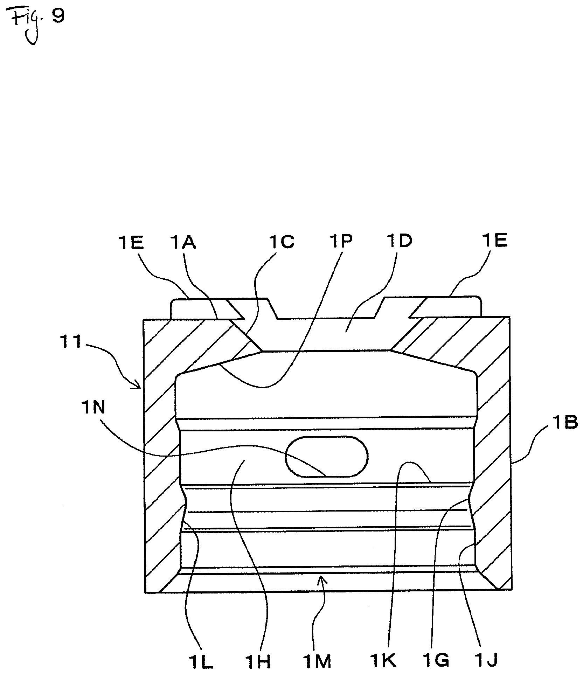

As shown in Fig. 8 and Fig. 9, in a cartridge cap 11 according to the second embodiment, a belt shaped receiving portion 1M is formed to continue in a circumferential direction on the inner periphery of the cylindrical wall portion 1B. The belt shaped receiving portion 1M is formed by changing the terrace shaped receiving portion 1F which is formed in the cartridge cap 1 according to the first embodiment so as to continue in a circumferential direction of the cylindrical wall portion 1B. A tucking portion 1G, a joint receiving portion 1H, a guide surface 1J, a locking portion 1K and a tapered guide surface 1L are formed in the belt shaped receiving portion 1M.

A pair of air relief holes 1N, 1N which pass through the cylindrical wall portion 1B in a radial direction are formed on the joint receiving surface 1H of the belt shaped receiving portion 1M. It is preferable to form the air relief holes 1N, 1N at the center portion or on the upper side of the vertical width of the joint receiving surface 1H. In this example shown in the drawing, they are formed in an oval shape of a size which is fit within the center portion of the vertical width of the joint receiving surface 1H, for example.

The opening area of the air relief holes 1N, 1N is set to 1/10 of the entire area of the joint receiving surface 1H or larger because enough air relief effect cannot be obtained if it is too small. The opening area is also set to 1/3 of the entire area of the joint receiving surface 1H or smaller because the strength of the cartridge cap 11 decreases if it is too large.

In the cartridge cap 11 according to the second embodiment, the inner surface of the top plate portion 1A is formed as a tapered top surface 1P in which the diameter decreases toward the upward direction in order to have a structure for improving the sealing performance by pushing the outer periphery of the inserted gasket 9 against the opening portion of the cylindrical cartridge 2 more tightly.

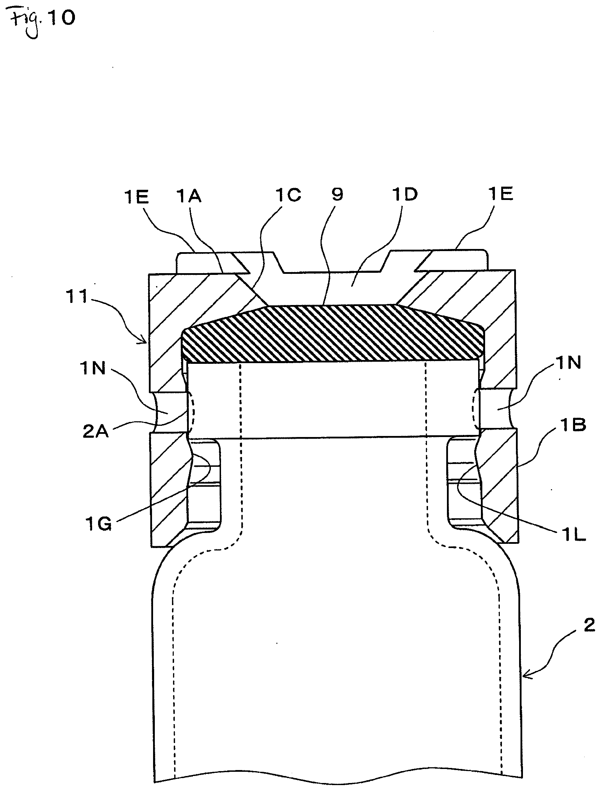

In the cartridge cap 11 according to the second embodiment configured as described above, as shown in Fig. 10, the cartridge cap 11 is to fit to the circular lip portion 2A which is formed at the opening portion of the cylindrical cartridge 2 by using a capper which is not shown in the drawing. By this capping operation, the cartridge cap 11 is attached to the head portion of the cylindrical cartridge 2 including the circular lip portion 2A and then the opening portion of the cylindrical cartridge 2 is sealed by the gasket 9 in this condition. That is to say, the cartridge cap 11 seals the opening portion of the cylindrical cartridge 2 by holding the circular lip portion 2A between the locking portion 1K of the belt shaped receiving portion 1M which is formed on the inner periphery of the cylindrical wall portion 1B and the tapered top surface 1P of the top plate portion 1A through the gasket 9.

The air which was sucked in the space between the cartridge cap 11 and the opening portion of the cylindrical cartridge 2 during a capping operation is released from the gap between the outer periphery of the circular lip portion 2A of the cylindrical cartridge 2 and the inner periphery of the cylindrical wall portion 1B of the cartridge cap 11 to the outside through a pair of air relief holes 1N, 1N which pass through the cylindrical wall portion 1B in a radial direction, as shown in Fig. 10.

Therefore, in the cartridge cap 11 according to the second embodiment, it is possible to prevent air from staying in the cylindrical cartridge 2 even if the air is sucked in by sealing the opening portion of the cylindrical cartridge 2 in which medicinal solution is filled.

Additionally, in the cartridge cap 11 according to the second embodiment, it is possible to more firmly seal the opening portion of the cylindrical cartridge 2 than the cartridge cap 1 according to the first embodiment because the belt shaped receiving portion 1M is formed continuously in a circumferential direction.

Furthermore, in the cartridge cap 11 according to the second embodiment, it is possible to firmly seal the opening portion of the cylindrical cartridge 2 by smoothly guiding the circular lip portion 2A to the holding position when the opening portion of the cylindrical cartridge 2 is sealed because the tucking portion 1G of the belt shaped receiving portion 1M which is formed on the inner periphery of the cylindrical wall portion 1B is formed in a belt shape which extends in a direction of the inner periphery of the cylindrical wall portion 1B and the tapered guide surface 1L is formed on the periphery to guide the outer periphery of the circular lip portion 2A of the cylindrical cartridge 2 to the side of the locking portion 1k which corresponds to the holding position.

A cartridge cap according to the present invention is limited to neither the aforementioned first embodiment nor the second embodiments. For example, the four terrace shaped receiving portion 1F, 1F,.. shown in Fig. 4 may be positioned in a circumferential direction of the cylindrical wall portion 1B at non-equiangular intervals which are a little bit shifted from equiangular intervals. It is also the case for the four thick portions 1E, 1E,.. shown in Fig. 4.

The terrace shaped receiving portions 1F, 1F,.. may be modified to three terrace shaped receiving portions 1F, 1F,.. which are positioned in a circumferential direction of the cylindrical wall portion 1B at equiangular intervals of 120 degrees, for example, or they may be positioned at non-equiangular intervals which are a little bit shifted from equiangular intervals. It is also the case for the four thick portions 1E, 1E,.. shown in Fig. 4.

Furthermore, the structure which is used to improve the sealing performance in the cartridge cap 11 according to the second embodiment, namely the structure wherein the inner surface of the top plate portion 1A is formed to decrease the diameter of the tapered top surface 1P in an upward direction can also be applied to the cartridge cap 1 according to the first embodiment.

### Explanation of the reference numbers

1: Cartridge cap
1A: Top plate portion
1B: Cylindrical wall portion
1C: Tapered wall surface
1D: Through hole
1E: Thick portion
1F: Terrace shaped receiving portion
1G: Tucking portion
1H: Joint receiving surface
1J: Guiding surface
1K: Locking portion
1L: Tapered guide surface
1M: Belt shaped receiving portion
1N: Air relief hole
1P: Tapered top surface
2: Cylindrical cartridge
2A: Circular lip portion
3: Piston
8: Gasket
9: Gasket
11: Cartridge cap

## Claims

1. A cartridge cap (1) for sealing the opening portion of a cylindrical cartridge (2) in which medicinal solution is filled, wherein a plurality of terrace shaped receiving portions (1F) are formed on the inner periphery of a cylindrical wall portion (1B) in a circumferential direction at predetermined intervals, the cylindrical wall portion (1B) continues to a top plate portion (1A), the terrace shaped receiving portion (1F) has a joint receiving surface (1H) for jointing with the outer periphery of a flange shaped lip portion (2A) formed at the opening portion of the cylindrical cartridge (2) and a tucking portion (1G) for holding the lip portion (2A) between the top plate portion (1A) and the tucking portion (1G), and an air relief passage (AR) is formed along the inner periphery of the cylindrical wall portion (1B) between the terrace shaped receiving portions (IF),
**characterized in that**
a plurality of thick portions (1E) are formed on the outer surface of the top plate portion (1A) at predetermined intervals in a circumferential direction, and the circumferential position of each thick portion (1E) is set so as to correspond to the circumferential position of each air relief passage (AR) between the terrace shaped receiving portions (1F).

2. A cartridge cap (1, 11) according to claim 1, wherein the tucking portion (1G) is formed in a belt shape which extends in a circumferential direction of the cylindrical wall portion (1B) and a tapered surface (1L) is formed on the periphery to guide the outer periphery of the lip portion (2A).

3. A cartridge cap (1, 11) according to claim 1 or 2, wherein a tapered top surface (1P) is formed on the inner surface of the top plate portion (1A) such that the diameter of the tapered top surface (1P) decreases in an upward direction toward the top plate portion (1A).

## Patentansprüche

1. Kartuschendeckel (1) zum Abdichten des Öffnungsabschnitts einer zylindrischen Kartusche (2), in die eine medizinische Lösung gefüllt ist, wobei eine Vielzahl von terrassenförmigen Aufnahmeabschnitten (1F) an dem Innenumfang des zylindrischen Wandabschnitts (1B) in einer Umfangsrichtung in vorbestimmten Abständen ausgebildet ist, der zylindrische Wandabschnitt (1B) sich zu einem oberen Plattenabschnitt (1A) fortsetzt, der terrassenförmige Aufnahmeabschnitt (1F) eine Verbindungsaufnahmefläche (1H) zum Verbinden mit dem Außenumfang eines flanschförmigen Lippenabschnitts (2A), der an dem Öffnungsabschnitt der zylindrischen Kartusche (2) ausgebildet ist, und einen Steckabschnitt (1G) zum Halten des Lippenabschnitts (2A) zwischen dem oberen Plattenabschnitt (1A) und dem Steckabschnitt (1G) hat, und ein Luftentlüftungsdurchgang (AR) entlang des Innenumfangs des zylindrischen Wandabschnitts (1B) zwischen den terrassenförmigen Aufnahmeabschnitten (1F) ausgebildet ist,
**dadurch gekennzeichnet, dass**
eine Vielzahl von dicken Abschnitten (1E) an der Außenfläche des oberen Wandabschnitts (1A) in vorbestimmten Abschnitten in einer Umfangsrichtung ausgebildet ist, und die Umfangsposition jedes dicken Abschnitts (1E) festgelegt ist, um zu der Umfangsposition jedes Luftentlüftungsdurchgangs (AR) zwischen den terrassenförmigen Aufnahmeabschnitten (1F) zu korrespondieren.

2. Kartuschendeckel (1, 11) nach Anspruch 1, wobei der Steckabschnitt (1G) in einer Bandform ausgebildet ist, die sich in einer Umfangsrichtung des zylindrischen Wandabschnitts (1B) erstreckt, und eine konische Fläche (1L) an dem Umfang ausgebildet ist, um den Außenumfang des Lippenabschnitts (2A) zu führen.

3. Kartuschendeckel (1, 11) nach Anspruch 1 oder 2, wobei eine konische obere Fläche (1P) an der Innenfläche des oberen Plattenabschnitts (1A) derart ausgebildet ist, dass sich der Durchmesser der konischen oberen Fläche (1P) in einer Aufwärtsrichtung zu dem oberen Plattenabschnitt (1A) hin verringert.

## Revendications

1. Capuchon de cartouche (1) pour sceller la partie d'ouverture d'une cartouche cylindrique (2) dans laquelle est versée une solution médicamenteuse, dans lequel une pluralité de parties de réception en forme de terrasse (1F) sont formées sur la périphérie interne d'une partie de paroi cylindrique (1B) dans une direction circonférentielle à intervalles prédéterminés, la partie de paroi cylindrique (1B) continue jusqu'à une partie de plaque supérieure (1A), la partie de réception en forme de terrasse (1F) a une surface de réception de joint (1H) pour jointer avec la périphérie externe d'une partie de lèvre en forme de bride (2A) formée au niveau de la partie d'ouverture de la cartouche cylindrique (2) et une partie de bordure (1G) pour maintenir la partie de lèvre (2A) entre la partie de plaque supérieure (1A) et la partie de bordure (1G), et un passage de décharge d'air (AR) est formé le long de la périphérie interne de la partie de paroi cylindrique (1B) entre les parties de réception en forme de terrasse (1F),
**caractérisé en ce que** :
une pluralité de parties épaisses (1E) sont formées sur la surface externe de la partie de plaque supérieure (1A) à intervalles prédéterminés dans une direction circonférentielle, et la position circonférentielle de chaque partie épaisse (1E) est déterminée afin de correspondre à la position circonférentielle de chaque passage de décharge d'air (AR) entre les parties de réception en forme de terrasse (1F).

2. Capuchon de cartouche (1, 11) selon la revendication 1, dans lequel la partie de bordure (1G) est formée selon une forme de courroie qui s'étend dans une direction circonférentielle de la partie de paroi cylindrique (1B) et une surface progressivement rétrécie (1L) est formée sur la périphérie pour guider la périphérie externe de la partie de lèvre (2A).

3. Capuchon de cartouche (1, 11) selon la revendication 1 ou 2, dans lequel une surface supérieure progressivement rétrécie (1P) est formée sur la surface interne de la partie de plaque supérieure (1A) de sorte que le diamètre de la surface supérieure progressivement rétrécie (1P) diminue dans une direction ascendante vers la partie de plaque supérieure (1A).
